# EUROPEAN PATENT APPLICATION

(11) **EP 4 759 809 A1**
(43) Date of publication of application: **17.06.2026**
(21) Application number: 24307115.6
(22) Date of filing: 16.12.2024
(51) Int. Cl.: C07D 417/12

(54) **SYNTHESIS OF LANIFIBRANOR**

(71) Applicant: Inventiva, 21121 Daix (FR)
(72) Inventor: BOUBIA, Benaissa, 21121 Daix (FR); GONZALEZ, Jérôme, 21121 Daix (FR); MOUNIER, Laurent, 21121 Daix (FR)
(74) Representative: Cabinet Beau de Loménie

(57) **Abstract**

The present disclosure is directed to a process for preparing lanifibranor comprising:
a) reacting 5-chloroindole with 2-(4-bromobutoxy)tetrahydropyran in a polar solvent;
b) reacting the compound obtained in step a) with 1,3-benzothiazole-6-sulfonyl chloride in a polar solvent;
c) deprotecting the compound obtained in step b);
d) oxidizing the compound obtained in step c).

## Description

### Field of the invention

The present invention relates to a process for preparing lanifibranor.

### Background of the invention

Lanifibranor or 1-(6-benzothiazolylsulfonyl)-5-chloro-1H-indole-2-butanoic acid is a pan-PPAR agonist which is currently in clinical development for the treatment of patients with non-alcoholic steatohepatitis (NASH).

Lanifibranor is described in example 117 of WO 2007/026097 and has the following structure:

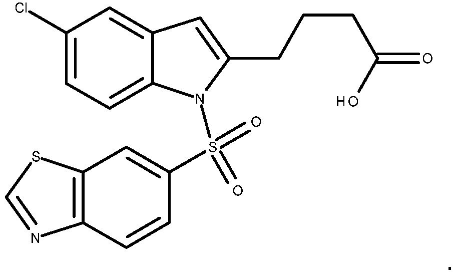

Its synthesis is described in WO 2007/026907 and in J Med Chem 2018, 61(6), 2246-2265. In the latter reference, the synthesis of lanifibranor comprises (i) reacting 4-chloro-2-iodoaniline with 1,3-benzothiazole-6-sulfonyl chloride to yield N-(4-chloro-2-iodoaniline)-1,3-benzothiazole-6-sulfonamide, (ii) reacting the compound obtained in step (i) with methyl hex-5-ynoate to yield methyl 4-[1-(1,3-benzothiazol-6-ylsulfonyl)-5-chloro-indol-2-yl]-butanoate, and (iii) deprotecting the ester group of the compound obtained in step (ii).

The present application aims at providing an alternative synthesis of lanifibranor.

### Summary of the invention

The present disclosure relates to a process for preparing lanifibranor of formula (I): 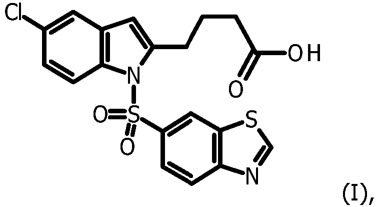 which comprises:
a) reacting 5-chloroindole with 2-(4-bromobutoxy)tetrahydropyran in a polar solvent to obtain 5-chloro-2-(4-tetrahydropyran-2-yloxybutyl)-1H-indole of formula (II): 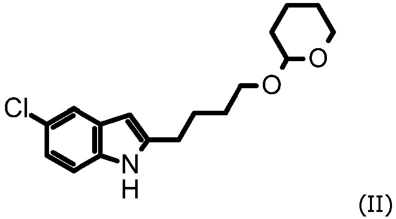
b) reacting 5-chloro-2-(4-tetrahydropyran-2-yloxybutyl)-1H-indole with 1,3-benzothiazole-6-sulfonyl chloride in a polar solvent to obtain 6-[5-chloro-2-(4-tetrahydropyran-2-yloxybutyl)indol-1-yl]sulfonyl-1,3-benzothiazole of formula (III): 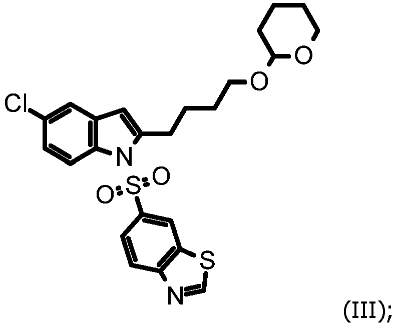
c) deprotecting the compound of formula (III) to obtain 4-[1-(1,3-benzothiazol-6-ylsulfonyl)-5-chloro-indol-2-yl]butan-1-ol of formula (IV): 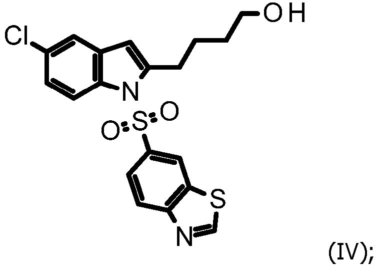 and
d) oxidizing the compound of formula (IV).

In some embodiments, step a) is carried out in the presence of a catalyst. In some embodiments, the catalyst is a palladium-based catalyst..

In some embodiments, step a) is carried out in the presence of a base.

In some embodiments, step a) is carried out at a temperature in the range from about 50°C to about 150°C.

In some embodiments, the same polar solvent is used in step a) and in step b).

### Description of the invention

As used herein, "room temperature" refers to a temperature of about 20°C ± 5°C. The present disclosure relates to a process for preparing lanifibranor of formula (I): 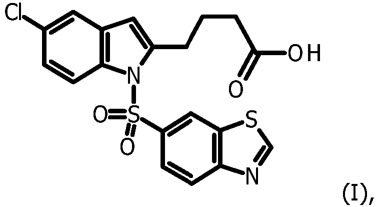 which comprises:
a) reacting 5-chloroindole with 2-(4-bromobutoxy)tetrahydropyran in a polar solvent to obtain 5-chloro-2-(4-tetrahydropyran-2-yloxybutyl)-1H-indole of formula (II): 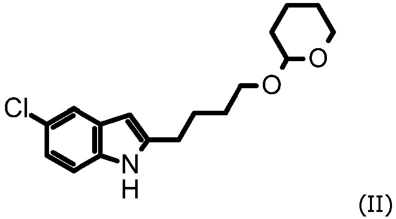
b) reacting 5-chloro-2-(4-tetrahydropyran-2-yloxybutyl)-1H-indole with 1,3-benzothiazole-6-sulfonyl chloride in a polar solvent to obtain 6-[5-chloro-2-(4-tetrahydropyran-2-yloxybutyl)indol-1-yl]sulfonyl-1,3-benzothiazole of formula (III): 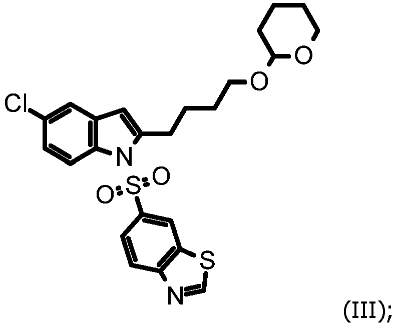
c) deprotecting the compound of formula (III) to obtain 4-[1-(1,3-benzothiazol-6-ylsulfonyl)-5-chloro-indol-2-yl]butan-1-ol of formula (IV): 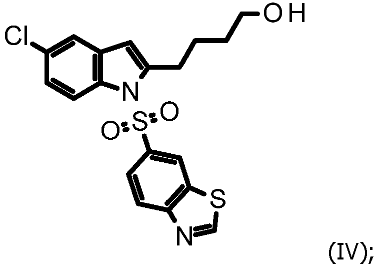 and
d) oxidizing the compound of formula (IV).

In some embodiments, step a) is carried out in the presence of a catalyst. In some embodiments, the catalyst is a palladium-based catalyst. In some embodiments, the palladium-based catalyst is is selected from bis(acetonitrile)dichloropalladium (II), palladium diacetate, bis(benzonitrile)dichloropalladium, palladium chloride, and palladium ditrifluoroacetate.

In some embodiments, step a) is carried out in the presence of from about 0.05 to about 1.0 equivalent of catalyst, based on the starting 5-chloroindole. In some embodiments, step a) is carried out in the presence of about 0.05 equivalent of catalyst. In some embodiments, step a) is carried out in the presence of about 0.1 equivalent of catalyst. In some embodiments, step a) is carried out in the presence of about 0.2 equivalent of catalyst. In some embodiments, step a) is carried out in the presence of about 0.3 equivalent of catalyst. In some embodiments, step a) is carried out in the presence of about 0.4 equivalent of catalyst. In some embodiments, step a) is carried out in the presence of about 0.5 equivalent of catalyst. In some embodiments, step a) is carried out in the presence of about 0.6 equivalent of catalyst. In some embodiments, step a) is carried out in the presence of about 0.7 equivalent of catalyst. In some embodiments, step a) is carried out in the presence of about 0.8 equivalent of catalyst. In some embodiments, step a) is carried out in the presence of about 0.9 equivalent of catalyst. In some embodiments, step a) is carried out in the presence of about 1.0 equivalent of catalyst.

In some embodiments, the coupling reaction is carried out in the presence of a base. Suitable bases include cesium carbonate, sodium carbonate, sodium hydrogencarbonate, potassium carbonate, sodium tert-butylate, potassium tert-butylate, sodium acetate, diisopropylamine, triethylamine, trans-diaminocyclohexane, 1,8-diazabicyclo[5.4.0]undec-7-enepyridine, 4-(dimethylamino)pyridine and mixtures thereof.

In some embodiments, step a) is carried out in the presence of from about 1.0 to about 5.0 equivalents of base, based on the starting 5-chloroindole.

In some embodiments, step a) is carried out in the presence of about 1.0 equivalent of base, based on the starting 5-chloroindole. In some embodiments, step a) is carried out in the presence of about 1.1 equivalents of base, based on the starting 5-chloroindole. In some embodiments, step a) is carried out in the presence of about 1.2 equivalents of base, based on the starting 5-chloroindole. In some embodiments, step a) is carried out in the presence of about 1.3 equivalents of base, based on the starting 5-chloroindole. In some embodiments, step a) is carried out in the presence of about 1.4 equivalents of base, based on the starting 5-chloroindole. In some embodiments, step a) is carried out in the presence of about 1.5 equivalents of base, based on the starting 5-chloroindole. In some embodiments, step a) is carried out in the presence of about 1.6 equivalents of base, based on the starting 5-chloroindole. In some embodiments, step a) is carried out in the presence of about 1.7 equivalents of base, based on the starting 5-chloroindole. In some embodiments, step a) is carried out in the presence of about 1.8 equivalents of base, based on the starting 5-chloroindole. In some embodiments, step a) is carried out in the presence of about 1.9 equivalents of base, based on the starting 5-chloroindole. In some embodiments, step a) is carried out in the presence of about 2.0 equivalents of base, based on the starting 5-chloroindole. In some embodiments, step a) is carried out in the presence of about 2.1 equivalents of base, based on the starting 5-chloroindole. In some embodiments, step a) is carried out in the presence of about 2.2 equivalents of base, based on the starting 5-chloroindole. In some embodiments, step a) is carried out in the presence of about 2.3 equivalents of base, based on the starting 5-chloroindole. In some embodiments, step a) is carried out in the presence of about 2.4 equivalents of base, based on the starting 5-chloroindole. In some embodiments, step a) is carried out in the presence of about 2.5 equivalents of base, based on the starting 5-chloroindole. In some embodiments, step a) is carried out in the presence of about 2.6 equivalents of base, based on the starting 5-chloroindole. In some embodiments, step a) is carried out in the presence of about 2.7 equivalents of base, based on the starting 5-chloroindole. In some embodiments, step a) is carried out in the presence of about 2.8 equivalents of base, based on the starting 5-chloroindole. In some embodiments, step a) is carried out in the presence of about 2.9 equivalents of base, based on the starting 5-chloroindole. In some embodiments, step a) is carried out in the presence of about 3.0 equivalents of base, based on the starting 5-chloroindole. In some embodiments, step a) is carried out in the presence of about 3.1 equivalents of base, based on the starting 5-chloroindole. In some embodiments, step a) is carried out in the presence of about 3.2 equivalents of base, based on the starting 5-chloroindole. In some embodiments, step a) is carried out in the presence of about 3.3 equivalents of base, based on the starting 5-chloroindole. In some embodiments, step a) is carried out in the presence of about 3.4 equivalents of base, based on the starting 5-chloroindole. In some embodiments, step a) is carried out in the presence of about 3.5 equivalents of base, based on the starting 5-chloroindole. In some embodiments, step a) is carried out in the presence of about 3.6 equivalents of base, based on the starting 5-chloroindole. In some embodiments, step a) is carried out in the presence of about 3.7 equivalents of base, based on the starting 5-chloroindole. In some embodiments, step a) is carried out in the presence of about 3.8 equivalents of base, based on the starting 5-chloroindole. In some embodiments, step a) is carried out in the presence of about 3.9 equivalents of base, based on the starting 5-chloroindole. In some embodiments, step a) is carried out in the presence of about 4.0 equivalents of base, based on the starting 5-chloroindole. In some embodiments, step a) is carried out in the presence of about 4.1 equivalents of base, based on the starting 5-chloroindole. In some embodiments, step a) is carried out in the presence of about 4.2 equivalents of base, based on the starting 5-chloroindole. In some embodiments, step a) is carried out in the presence of about 4.3 equivalents of base, based on the starting 5-chloroindole. In some embodiments, step a) is carried out in the presence of about 4.4 equivalents of base, based on the starting 5-chloroindole. In some embodiments, step a) is carried out in the presence of about 4.5 equivalents of base, based on the starting 5-chloroindole. In some embodiments, step a) is carried out in the presence of about 4.6 equivalents of base, based on the starting 5-chloroindole. In some embodiments, step a) is carried out in the presence of about 4.7 equivalents of base, based on the starting 5-chloroindole. In some embodiments, step a) is carried out in the presence of about 4.8 equivalents of base, based on the starting 5-chloroindole. In some embodiments, step a) is carried out in the presence of about 4.9 equivalents of base, based on the starting 5-chloroindole. In some embodiments, step a) is carried out in the presence of about 5.0 equivalents of base, based on the starting 5-chloroindole.

In some embodiments, step a) is carried out at a temperature in the range from about 50°C to about 150°C. In some embodiments, step a) is carried out at a temperature in the range from about 50°C to about 145°C. In some embodiments, step a) is carried out at a temperature in the range from about 50°C to about 140°C. In some embodiments, step a) is carried out at a temperature in the range from about 50°C to about 135°C. In some embodiments, step a) is carried out at a temperature in the range from about 50°C to about 130°C. In some embodiments, step a) is carried out at a temperature in the range from about 50°C to about 125°C. In some embodiments, step a) is carried out at a temperature in the range from about 50°C to about 120°C. In some embodiments, step a) is carried out at a temperature in the range from about 50°C to about 115°C. In some embodiments, step a) is carried out at a temperature in the range from about 50°C to about 110°C. In some embodiments, step a) is carried out at a temperature in the range from about 50°C to about 105°C. In some embodiments, step a) is carried out at a temperature in the range from about 50°C to about 100°C. In some embodiments, step a) is carried out at a temperature in the range from about 50°C to about 95°C. In some embodiments, step a) is carried out at a temperature in the range from about 50°C to about 90°C. In some embodiments, step a) is carried out at a temperature in the range from about 50°C to about 85°C. In some embodiments, step a) is carried out at a temperature in the range from about 50°C to about 80°C. In some embodiments, step a) is carried out at a temperature in the range from about 55°C to about 80°C. In some embodiments, step a) is carried out at a temperature in the range from about 60°C to about 80°C. In some embodiments, step a) is carried out at a temperature in the range from about 65°C to about 75°C.

In some embodiments, step a) is carried out at a temperature of about 50°C. In some embodiments, step a) is carried out at a temperature of about 55°C, for example of about 56°C, for example of about 57°C, for example of about 58°C, for example of about 59°C, for example of about 60°C, for example of about 61°C, for example of about 62°C, for example of about 63°C, for example of about 64°C, for example of about 65°C, for example of about 66°C, for example of about 67°C, for example of about 68°C, for example of about 69°C, for example of about 70°C, for example of about 71°C, for example of about 72°C, for example of about 73°C, for example of about 74°C, for example of about 75°C, for example of about 76°C, for example of about 77°C, for example of about 78°C, for example of about 79°C, for example of about 80°C, for example of about 81°C, for example of about 82°C, for example of about 83°C, for example of about 84°C, for example of about 85°C, for example of about 86°C, for example of about 87°C, for example of about 88°C, for example of about 89°C, for example of about 90°C, for example of about 91°C, for example of about 92°C, for example of about 93°C, for example of about 94°C, for example of about 95°C, for example of about 96°C, for example of about 97°C, for example of about 98°C, for example of about 99°C, for example of about 100°C, for example of about 101°C, for example of about 102°C, for example of about 103°C, for example of about 104°C, for example of about 105°C, for example of about 106°C, for example of about 107°C, for example of about 108°C, for example of about 109°C, for example of about 110°C, for example of about 111°C, for example of about 112°C, for example of about 113°C, for example of about 114°C, for example of about 115°C, for example of about 116°C, for example of about 117°C, for example of about 118°C, for example of about 119°C, for example of about 120°C, for example of about 121°C, for example of about 122°C, for example of about 123°C, for example of about 124°C, for example of about 125°C, for example of about 126°C, for example of about 127°C, for example of about 128°C, for example of about 129°C, for example of about 130°C, for example of about 131°C, for example of about 132°C, for example of about 133°C, for example of about 134°C, for example of about 135°C, for example of about 136°C, for example of about 137°C, for example of about 138°C, for example of about 139°C, for example of about 140°C, for example of about 141°C, for example of about 142°C, for example of about 143°C, for example of about 144°C, for example of about 145°C, for example of about 146°C, for example of about 147°C, for example of about 148°C, for example of about 149°C, for example of about 150°C. In some embodiments, step a) is carried out at a temperature of about 60°C. In some embodiments, step a) is carried out at a temperature of about 65°C. In some embodiments, step a) is carried out at a temperature of about 70°C. In some embodiments, step a) is carried out at a temperature of about 75°C. In some embodiments, step a) is carried out at a temperature of about 80°C. In some embodiments, step a) is carried out at a temperature of about 85°C. In some embodiments, step a) is carried out at a temperature of about 90°C. In some embodiments, step a) is carried out at a temperature of about 95°C. In some embodiments, step a) is carried out at a temperature of about 100°C. In some embodiments, step a) is carried out at a temperature of about 105°C. In some embodiments, step a) is carried out at a temperature of about 110°C. In some embodiments, step a) is carried out at a temperature of about 115°C. In some embodiments, step a) is carried out at a temperature of about 120°C. In some embodiments, step a) is carried out at a temperature of about 125°C. In some embodiments, step a) is carried out at a temperature of about 130°C. In some embodiments, step a) is carried out at a temperature of about 135°C. In some embodiments, step a) is carried out at a temperature of about 140°C. In some embodiments, step a) is carried out at a temperature of about 145°C. In some embodiments, step a) is carried out at a temperature of about 150°C.

In some embodiments, step a) is carried out for at least about 1 hour and up to about 72 hours, such as at least about 1 hours, at least about 1.5 hours, at least about 2 hours, at least about 2.5 hours, at least about 3 hours, at least about 3.5 hours, at least about 4 hours, at least about 4.5 hours, at least about 5 hours, at least about 5.5 hours, at least about 6 hours, at least about 6.5 hours, at least about 7 hours, at least about 7.5 hours, at least about 8 hours, at least about 8.5 hours, at least about 9 hours, at least about 9.5 hours, at least about 10 hours, at least about 10.5 hours, at least about 11 hours, at least about 11.5 hours, at least about 12 hours, at least about 12.5 hours, at least about 13 hours, at least about 13.5 hours, at least about 14 hours, at least about 14.5 hours, at least about 15 hours, at least about 15.5 hours, at least about 16 hours, at least about 16.5 hours, at least about 17 hours, at least about 17.5 hours, at least about 18 hours, at least about 18.5 hours, at least about 19 hours, at least about 19.5 hours, at least about 20 hours, at least about 20.5 hours, at least about 21 hours, at least about 21.5 hours, at least about 22 hours, at least about 22.5 hours, at least about 23 hours, at least about 23.5 hours, at least about 24 hours, at least about 24.5 hours, at least about 25 hours, at least about 25.5 hours, at least about 26 hours, at least about 26.5 hours, at least about 27 hours, at least about 27.5 hours, at least about 28 hours, at least about 28.5 hours, at least about 29 hours, at least about 29.5 hours, at least about 30 hours, at least about 30.5 hours, at least about 31 hours, at least about 31.5 hours, at least about 32 hours, at least about 32.5 hours, at least about 33 hours, at least about 33.5 hours, at least about 34 hours, at least about 34.5 hours, at least about 35 hours, at least about 35.5 hours, at least about 36 hours, at least about 36.5 hours, at least about 37 hours, at least about 37.5 hours, at least about 38 hours, at least about 38.5 hours, at least about 39 hours, at least about 39.5 hours, at least about 40 hours, at least about 40.5 hours, at least about 41 hours, at least about 41.5 hours, at least about 42 hours, at least about 42.5 hours, at least about 43 hours, at least about 43.5 hours, at least about 44 hours, at least about 44.5 hours, at least about 45 hours, at least about 45.5 hours at least about 46 hours, at least about 46.5 hours, at least about 47 hours, at least about 47.5 hours at least about 48 hours, at least about 48.5 hours, at least about 49 hours, at least about 49.5 hours, at least about 50 hours, at least about 50.5 hours, at least about 51 hours, at least about 51.5 hours, at least about 52 hours, at least about 52.5 hours, at least about 53 hours, at least about 53.5 hours, at least about 54 hours, at least about 54.5 hours, at least about 55 hours, at least about 55.5 hours, at least about 56 hours, at least about 56.5 hours, at least about 57 hours, at least about 57.5 hours, at least about 58 hours, at least about 58.5 hours, at least about 59 hours, at least about 59.5 hours, at least about 60 hours, at least about 60.5 hours, at least about 61 hours, at least about 61.5 hours, at least about 62 hours, at least about 62.5 hours, at least about 63 hours, at least about 63.5 hours, at least about 64 hours, at least about 64.5 hours, at least about 65 hours, at least about 65.5 hours, at least about 66 hours, at least about 66.5 hours, at least about 67 hours, at least about 67.5 hours, at least about 68 hours, at least about 68.5 hours, at least about 69 hours, at least about 69.5 hours, at least about 70 hours, at least about 70.5 hours, at least about 71 hours, at least about 71.5 hours, at least about 72 hours.

In some embodiments, step a) is carried out for no more than about 72 hours, such as no more than about 71.5 hours, no more than about 71 hours, no more than about 70.5 hours, no more than about 70 hours, no more than about 69.5 hours, no more than about 69 hours, no more than about 68.5 hours, no more than about 68 hours, no more than about 67.5 hours, no more than about 67 hours, no more than about 66.5 hours, no more than about 66 hours, no more than about 65.5 hours, no more than about 65 hours, no more than about 64.5 hours, no more than about 64 hours, no more than about 63.5 hours, no more than about 63 hours, no more than about 62.5 hours, no more than about 62 hours, no more than about 61.5 hours, no more than about 61 hours, no more than about 60.5 hours, no more than about 60 hours, no more than about 59.5 hours, no more than about 59 hours, no more than about 58.5 hours, no more than about 58 hours, no more than about 57.5 hours, no more than about 57 hours, no more than about 56.5 hours, no more than about 56 hours, no more than about 55.5 hours, no more than about 55 hours, no more than about 54.5 hours, no more than about 54 hours, no more than about 53.5 hours, no more than about 53 hours, no more than about 52.5 hours, no more than about 52 hours, no more than about 51.5 hours, no more than about 51 hours, no more than about 50.5 hours, no more than about 50 hours, no more than about 49.5 hours, no more than about 49 hours, no more than about 48.5 hours, no more than about 48 hours, no more than about 47.5 hours, no more than about 47 hours, no more than about 46.5 hours, no more than about 46 hours, no more than about 45.5 hours, no more than about 45 hours, no more than about 44.5 hours, no more than about 44 hours, no more than about 43.5 hours, no more than about 43 hours, no more than about 42.5 hours, no more than about 31 hours, no more than about 41.5 hours, no more than about 41 hours, no more than about 40.5 hours, no more than about 40 hours, no more than about 39.5 hours, no more than about 39 hours, no more than about 38.5 hours, no more than about 38 hours, no more than about 37.5 hours, no more than about 37 hours, no more than about 36.5 hours, no more than about 36 hours, no more than about 35.5 hours, no more than about 35 hours, no more than about 34.5 hours, no more than about 34 hours, no more than about 33.5 hours, no more than about 33 hours, no more than about 32.5 hours, no more than about 32 hours, no more than about 31.5 hours, no more than about 31 hours, no more than about 30.5 hours, no more than about 30 hours, no more than about 29.5 hours, no more than about 29 hours, no more than about 28.5 hours, no more than about 28 hours, no more than about 27.5 hours, no more than about 27 hours, no more than about 26.5 hours, no more than about 26 hours, no more than about 25.5 hours, no more than about 25 hours, no more than about 24.5 hours, or no more than about 24 hours.

In some embodiments, the polar solvent used in step a) is selected from ethyl acetate, acetonitrile, an ether such as methyltetrahydrofuran or dioxane, a water/ether mixture, a water/methanol mixture, toluene, pyridine, sulfolane, tetrahydrothiophene 1,1-dioxyde, N-methyl-2-pyrrolidone (NMP), 1,3-dimethyl-3,4,5,6-tetrahydro-2(1H)-pyrimidinone (DMPU), dimethylsulfoxide and dimethylformamide.

In some embodiments, step b) is carried out in the presence of from about 0.1 to about 5.0 equivalents of 1,3-benzothiazole-6-sulfonyl chloride, based on the starting 5-chloro-2-(4-tetrahydropyran-2-yloxybutyl)-1H-indole. In some embodiments, step b) is carried out in the presence of about 0.1 equivalent of 1,3-benzothiazole-6-sulfonyl chloride. In some embodiments, step b) is carried out in the presence of about 0.2 equivalent of 1,3-benzothiazole-6-sulfonyl chloride. In some embodiments, step b) is carried out in the presence of about 0.3 equivalent of 1,3-benzothiazole-6-sulfonyl chloride. In some embodiments, step b) is carried out in the presence of about 0.4 equivalent of 1,3-benzothiazole-6-sulfonyl chloride. In some embodiments, step b) is carried out in the presence of about 0.5 equivalent of 1,3-benzothiazole-6-sulfonyl chloride. In some embodiments, step b) is carried out in the presence of about 0.6 equivalent of 1,3-benzothiazole-6-sulfonyl chloride. In some embodiments, step b) is carried out in the presence of about 0.7 equivalent of 1,3-benzothiazole-6-sulfonyl chloride. In some embodiments, step b) is carried out in the presence of about 0.8 equivalent of 1,3-benzothiazole-6-sulfonyl chloride. In some embodiments, step b) is carried out in the presence of about 0.9 equivalent of 1,3-benzothiazole-6-sulfonyl chloride. In some embodiments, step b) is carried out in the presence of about 1.0 equivalent of 1,3-benzothiazole-6-sulfonyl chloride. In some embodiments, step b) is carried out in the presence of about 1.1 equivalents of 1,3-benzothiazole-6-sulfonyl chloride. In some embodiments, step b) is carried out in the presence of about 1.2 equivalents of 1,3-benzothiazole-6-sulfonyl chloride. In some embodiments, step b) is carried out in the presence of about 1.3 equivalents of 1,3-benzothiazole-6-sulfonyl chloride. In some embodiments, step b) is carried out in the presence of about 1.4 equivalents of 1,3-benzothiazole-6-sulfonyl chloride. In some embodiments, step b) is carried out in the presence of about 1.5 equivalents of 1,3-benzothiazole-6-sulfonyl chloride. In some embodiments, step b) is carried out in the presence of about 1.6 equivalents of 1,3-benzothiazole-6-sulfonyl chloride. In some embodiments, step b) is carried out in the presence of about 1.7 equivalents of 1,3-benzothiazole-6-sulfonyl chloride. In some embodiments, step b) is carried out in the presence of about 1.8 equivalents of 1,3-benzothiazole-6-sulfonyl chloride. In some embodiments, step b) is carried out in the presence of about 1.9 equivalents of 1,3-benzothiazole-6-sulfonyl chloride. In some embodiments, step b) is carried out in the presence of about 2.0 equivalents of 1,3-benzothiazole-6-sulfonyl chloride. In some embodiments, step b) is carried out in the presence of about 2.1 equivalents of 1,3-benzothiazole-6-sulfonyl chloride. In some embodiments, step b) is carried out in the presence of about 2.2 equivalents of 1,3-benzothiazole-6-sulfonyl chloride. In some embodiments, step b) is carried out in the presence of about 2.3 equivalents of 1,3-benzothiazole-6-sulfonyl chloride. In some embodiments, step b) is carried out in the presence of about 2.4 equivalents of 1,3-benzothiazole-6-sulfonyl chloride. In some embodiments, step b) is carried out in the presence of about 2.5 equivalents of 1,3-benzothiazole-6-sulfonyl chloride. In some embodiments, step b) is carried out in the presence of about 2.6 equivalents of 1,3-benzothiazole-6-sulfonyl chloride. In some embodiments, step b) is carried out in the presence of about 2.7 equivalents of 1,3-benzothiazole-6-sulfonyl chloride. In some embodiments, step b) is carried out in the presence of about 2.8 equivalents of 1,3-benzothiazole-6-sulfonyl chloride. In some embodiments, step b) is carried out in the presence of about 2.9 equivalents of 1,3-benzothiazole-6-sulfonyl chloride. In some embodiments, step b) is carried out in the presence of about 3.0 equivalents of 1,3-benzothiazole-6-sulfonyl chloride. In some embodiments, step b) is carried out in the presence of about 3.1 equivalents of 1,3-benzothiazole-6-sulfonyl chloride. In some embodiments, step b) is carried out in the presence of about 3.2 equivalents of 1,3-benzothiazole-6-sulfonyl chloride. In some embodiments, step b) is carried out in the presence of about 3.3 equivalents of 1,3-benzothiazole-6-sulfonyl chloride. In some embodiments, step b) is carried out in the presence of about 3.4 equivalents of 1,3-benzothiazole-6-sulfonyl chloride. In some embodiments, step b) is carried out in the presence of about 3.5 equivalents of 1,3-benzothiazole-6-sulfonyl chloride. In some embodiments, step b) is carried out in the presence of about 3.6 equivalents of 1,3-benzothiazole-6-sulfonyl chloride. In some embodiments, step b) is carried out in the presence of about 3.7 equivalents of 1,3-benzothiazole-6-sulfonyl chloride. In some embodiments, step b) is carried out in the presence of about 3.8 equivalents of 1,3-benzothiazole-6-sulfonyl chloride. In some embodiments, step b) is carried out in the presence of about 3.9 equivalents of 1,3-benzothiazole-6-sulfonyl chloride. In some embodiments, step b) is carried out in the presence of about 4.0 equivalents of 1,3-benzothiazole-6-sulfonyl chloride. In some embodiments, step b) is carried out in the presence of about 4.1 equivalents of 1,3-benzothiazole-6-sulfonyl chloride. In some embodiments, step b) is carried out in the presence of about 4.2 equivalents of 1,3-benzothiazole-6-sulfonyl chloride. In some embodiments, step b) is carried out in the presence of about 4.3 equivalents of 1,3-benzothiazole-6-sulfonyl chloride. In some embodiments, step b) is carried out in the presence of about 4.4 equivalents of 1,3-benzothiazole-6-sulfonyl chloride. In some embodiments, step b) is carried out in the presence of about 4.5 equivalents of 1,3-benzothiazole-6-sulfonyl chloride. In some embodiments, step b) is carried out in the presence of about 4.6 equivalents of 1,3-benzothiazole-6-sulfonyl chloride. In some embodiments, step b) is carried out in the presence of about 4.7 equivalents of 1,3-benzothiazole-6-sulfonyl chloride. In some embodiments, step b) is carried out in the presence of about 4.8 equivalents of 1,3-benzothiazole-6-sulfonyl chloride. In some embodiments, step b) is carried out in the presence of about 4.9 equivalents of 1,3-benzothiazole-6-sulfonyl chloride. In some embodiments, step b) is carried out in the presence of about 5.0 equivalents of 1,3-benzothiazole-6-sulfonyl chloride.

In some embodiments, step b) is carried out at room temperature or below room temperature. In some embodiments, step b) is carried out at a temperature of about 20°C or less, such as about 15°C or less, about 10°C or less, about 5°C or less, or about 0°C. In some embodiments step b) is carried out at a temperature no lower than -5°C.

In some embodiments, step b) is carried out for at least about 30 minutes and up to about 36 hours, such as at least about 30 minutes, at least about 1 hour, at least about 1.5 hours, at least about 2 hours, at least about 2.5 hours, at least about 3 hours, at least about 3.5 hours, at least about 4 hours, at least about 4.5 hours, at least about 5 hours, at least about 5.5 hours, at least about 6 hours, at least about 6.5 hours, at least about 7 hours, at least about 7.5 hours, at least about 8 hours, at least about 8.5 hours, at least about 9 hours, at least about 9.5 hours, at least about 10 hours, at least about 10.5 hours, at least about 11 hours, at least about 11.5 hours, at least about 12 hours, at least about 12.5 hours, at least about 13 hours, at least about 13.5 hours, at least about 14 hours, at least about 14.5 hours, at least about 15 hours, at least about 15.5 hours, at least about 16 hours, at least about 16.5 hours, at least about 17 hours, at least about 17.5 hours, or at least about 18 hours. In some embodiments, step b) is carried out for no more than about 36 hours, such as no more than about 35.5 hours, no more than about 35 hours, no more than about 34.5 hours, no more than about 34 hours, no more than about 33.5 hours, no more than about 33 hours, no more than about 32.5 hours, no more than about 32 hours, no more than about 31.5 hours, no more than about 31 hours, no more than about 30.5 hours, no more than about 30 hours, no more than about 29.5 hours, no more than about 29 hours, no more than about 28.5 hours, no more than about 28 hours, no more than about 27.5 hours, no more than about 27 hours, no more than about 26.5 hours, no more than about 26 hours, no more than about 25.5 hours, no more than about 25 hours, no more than about 24.5 hours, or no more than about 24 hours.

In some embodiments, the polar solvent used in step b) is selected from an ether such as methyltetrahydrofuran or dioxane, toluene, pyridine, N-methyl-2-pyrrolidone, 1,3-dimethyl-3,4,5,6-tetrahydro-2(1H)-pyrimidinone, dimethylsulfoxide and dimethylformamide.

In some embodiments, the same polar solvent is used in step a) and in step b).

Deprotection of the tetrahydropyran ether group of formula (III) is carried out in step c) by techniques well known in the art, for example by reacting the compound of formula (III) with p-toluenesulfonic acid in methanol. In some embodiments, step c) is carried out in the presence of from about 0.01 to about 0.5 equivalent of p-toluenesulfonic acid, based on the starting 6-[5-chloro-2-(4-tetrahydropyran-2-yloxybutyl)indol-1-yl]sulfonyl-1,3-benzothiazole.

In some embodiments, step c) is carried ou at a temperature from about 15°C to about 30°C. In some embodiments, step c) is carried out for at least 1 hour and up to about 24 hours.

Oxidation of the compound of formula (IV) is carried out in step d) by techniques well known in the art, for example by reacting the alcohol of formula (IV) with an oxidizing agent, such as chromium trioxide, in a polar solvent. In some embodiments, step d) is carried out in the presence of from about 0.5 to about 2 equivalents of oxidizing agent, based on the starting 4-[1-(1,3-benzothiazol-6-ylsulfonyl)-5-chloro-indol-2-yl]butan-1-ol.

In some embodiments, the polar solvent used in step d) is selected from ethyl acetate, dichloromethane, tetrahydrofuran, acetonitrile, acetone, dimethylsulfoxide and dimethylformamide.

In some embodiments, step d) is carried ou at a temperature of no more than about 15°C, for example no more than about 10°C, no more than about 5°C, e.g. about 0°C. In some embodiments step d) is carried out at a temperature no lower than -5°C.

In some embodiments, step d) is carried out for at least about 15 minutes and up to about 2 hours.

The invention is illustrated by the following, non-limiting examples.

### Examples

NMR spectra were obtained using Bruker 400MHz.

### Abbreviations

AcOH = acetic acid(CH₃CN)₂PdCl₂ = Bis(acetonitrile)dichloropalladium (II)
br s = broad singulet
CV = column volume
d = doublet
DCM = dichloromethane
dd =doublet of doublet
DMA = dimethylacetamide
DMF = N,N-dimethylformamide
DMSO = dimethylsulfoxide
DMSO-d6 = deuteriated DMSO
dt = doublet of triplet
eq = equivalent
Et₂O = ethyl ether
EtOAc = ethyl acetate
g = gram(s)
h = Hour(s)
m = multiplet
MeOH = methanol
mL = milliliter
NaH = sodium hydride
NaHCO₃ = sodium hydrogencarbonate
NMR = Nuclear Magnetic Resonance
ppm = parts per million
quint =quintuplet
RT = room temperature
s = singulet
TFA = trifluoroacetic acid

### Example 1: 5-chloro-2-(4-tetrahydropyran-2-yloxybutyl)-1H-indole

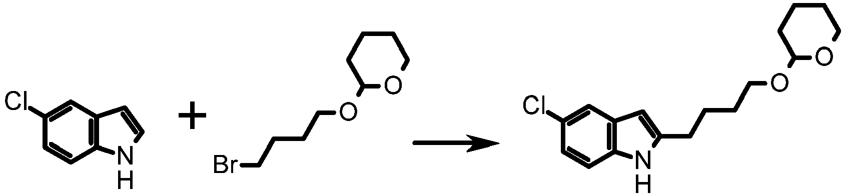

5-chloroindole (1 eq; 1.25 g), 2-(4-bromobutoxy)tetrahydropyran (1.7 eq; 3.32 g), (CH₃CN)₂PdCl₂ (0.10 eq; 0.214 g), norbornylene (2 eq; 1.55 g) and NaHCO₃ (2 eq; 1.39 g) were mixed in DMA (50 mL) and water (0.50 mL) and the suspension was bubbled with N₂ for 5 min. It was heated at 70°C for 21h, and then further heated at 90°C for 20h to complete the reaction. After cooling, water and Et₂O were added, the organic layer was washed twice with water, dried over MgSO₄, filtered and evaporated to afford 5 g of a dark oil. The oil was purified on a 100g silicagel cartridge eluting from 10% to 30% EtOAc in cyclohexane to afford the title compound as a brown oil after concentration of the desired fractions (1.18 g; yield: 46 %).

¹H NMR (400 MHz, DMSO-*d*6) δ ppm 1.09 (t, *J*=7.0 Hz, 1 H) ; 1.38 - 1.51 (m, 4 H) ; 1.53 - 1.65 (m, 2 H); 1.66 - 1.79 (m, 2 H); 2.73 (t, *J*=7.5 Hz, 2 H); 3.32 - 3.44 (m, 3 H); 3.61 - 3.75 (m, 2 H); 4.53 (t, *J*=3.5 Hz, 1 H); 6.13 (dd, *J*=2.0, 0.77 Hz, 1 H); 6.97 (dd, *J*=8.6, 2.1 Hz, 1 H); 7.26 (dt, *J*=8.5, 0.70 Hz, 1 H); 7.43 (d, *J*=2.1 Hz, 1 H); 11.09 (br s, 1 H). APCI MS m/z 308 [M+H]+; UPLC-MS (210-260 nm) purity = 100%.

### Example 2: 6-[5-chloro-2-(4-tetrahydropyran-2-yloxybutyl)indol-1-yl]sulfonyl-1,3-benzothiazole

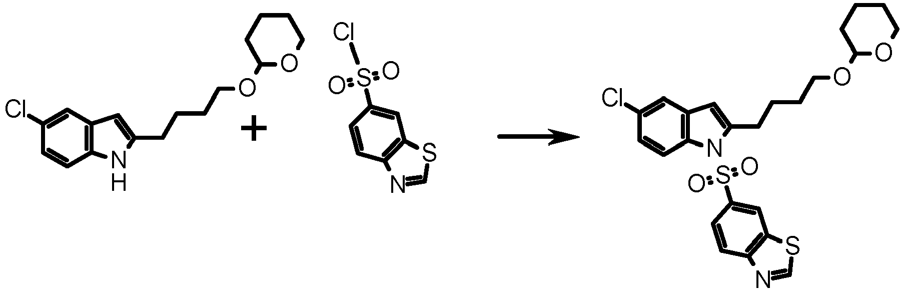

At 0°C, under N₂, NaH 60% in oil (1.5 eq; 0.23 g) was added to a solution of 5-chloro-2-(4-tetrahydropyran-2-yloxybutyl)-1H-indole (1 eq; 1.18 g) in DMF (11.80 mL) and the brown suspension was stirred 5 min at 0°C. 1,3-benzothiazole-6-sulfonyl chloride (1.2 eq; 1.08 g) was added portionwise at 0°C and the reaction mixture was stirred from 0°C to RT over 30 min. After cooling, water and EtzO were added, the organic layer was washed twice with water, dried over MgSO₄, filtered and evaporated. It was purified on a 25g silicagel cartridge eluting from 15 to 40% EtOAc in cyclohexane to afford the title compound as a yellow oil after concentration of the desired fractions (0.73 g; yield: 38 %).

1H NMR (400 MHz, DMSO-*d*6) δ ppm 1.38 - 1.49 (m, 3 H); 1.55 - 1.80 (m, 7 H), 3.07 (t, *J*=7.5 Hz, 2 H); 3.32 - 3.43 (m, 2 H); 3.61 - 3.75 (m, 2 H); 4.51 - 4.54 (m, 1 H); 6.61 (d, *J*=0.7 Hz, 1 H); 7.31 (dd, *J*=8.9, 2.2 Hz, 1 H); 7.57 (d, *J*=1.9 Hz, 1 H); 7.83 (dd, *J*=8.8, 2.0 Hz, 1 H); 8.09 (d, J=9.0 Hz, 1 H); 8.20 (d, *J*=8.8 Hz, 1 H); 8.98 (d, *J*=1.7 Hz, 1 H); 9.65 (s, 1 H).

### Example 3: 4-[1-(1,3-benzothiazol-6-ylsulfonyl)-5-chloro-indol-2-yl]butan-1-ol

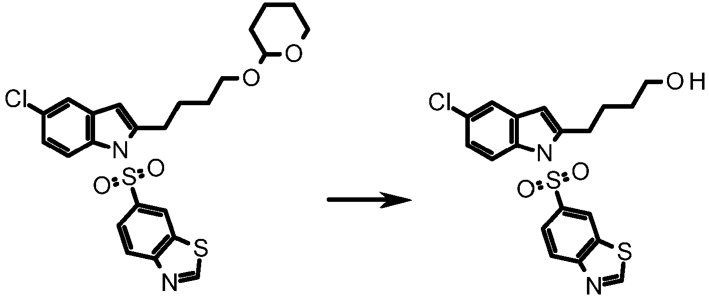

p-toluenesulfonic acid hydrate (0.05 eq; 0.014g) was added to a solution of 6-[5-chloro-2-(4-tetrahydropyran-2-yloxybutyl)indol-1-yl]sulfonyl-1,3-benzothiazole (1 eq; 0.73 g) in MeOH (7.30 mL) and the reaction mixture was stirred at RT for 18h. A beige precipitate appeared. It was filtered to afford 0.37 g of a solid. The filtrate was concentrated, a precipitate appeared, which was filtered, this operation being repeated twice. The three solids were combined to afford the title compound as a beige powder (0.45 g; yield: 74%).

¹H NMR (400 MHz, DMSO-d6) δ ppm 1.49 - 1.58 (m, 2 H); 1.69 - 1.78 (m, 2 H); 3.05 (t, J=7.3 Hz, 2 H); 3.41 - 3.46 (m, 2 H); 4.42 (t, J=5.2 Hz, 1 H); 6.60 (d, J=0.7 Hz, 1 H); 7.29 - 7.33 (m, 1 H); 7.57 (d, J=1.9 Hz, 1 H); 7.82 - 7.85 (m, 1 H); 8.09 (d, J=8.9 Hz, 1 H); 8.20 (d, J=8.7 Hz, 1 H); 8.98 (d, J=1.8 Hz, 1 H); 9.65 (s, 1 H).

APCI MS m/z 421 [M+H]+; UPLC-MS (210-260 nm) purity = 100%.

### Example 4: 4-[1-(1,3-benzothiazol-6-ylsulfonyl)-5-chloro-indol-2-yl]butanoic acid

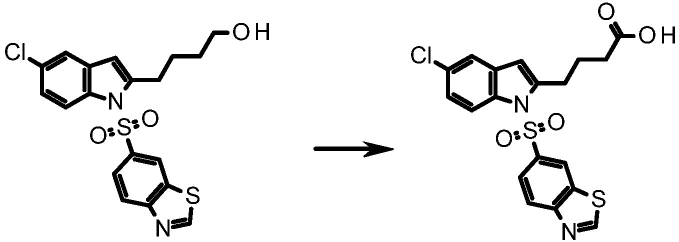

At 0°C, chromium trioxide (1.1 eq; 0.59 mL; 2.00 mol/L) was added to 4-[1-(1,3-benzothiazol-6-ylsulfonyl)-5-chloro-indol-2-yl]butan-1-ol (1 eq; 0.45 g) in acetone (4.50 mL) and the reaction mixture was stirred for 30 min at 0°C. The mixture was diluted with EtOAc and washed with a saturated solution of NH₄Cl. The organic layer was dried over MgSO4, filtered and evaporated. 1.3 ml of AcOH were added, the solution was heated with a heat gun and allowed to cool down overnight to afford a solid which was filtered to afford the title compound as a pale-yellow powder (0.13 g; yield: 29%).

¹H NMR (400 MHz, DMSO-d6) δ ppm 1.92 - 2.00 (m, 2 H); 2.36 (t, J=7.4 Hz, 2 H); 3.09 (t, J=7.4 Hz, 2 H); 6.62 (s, 1 H); 7.32 (dd, J=8.9, 2.2 Hz, 1 H); 7.57 (d, J=1.9 Hz, 1 H); 7.85 (dd, J=8.8, 2.1 Hz, 1 H); 8.09 (d, J=8.9 Hz, 1 H); 8.20 (d, J=8.7 Hz, 1 H); 8.97 (d, J=1.7 Hz, 1 H); 9.65 (s, 1 H); 12.03 -12.15 (m, 1 H).

APCI MS m/z 435 [M+H]+; UPLC-MS (210-260 nm) purity = 95%.

Aspects of the present disclosure are further illustrated by reference to the following, non-limiting embodiments.
1. A process for preparing lanifibranor of formula (I): 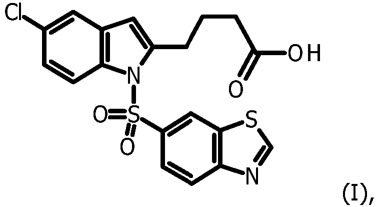 which comprises:
   a) reacting 5-chloroindole with 2-(4-bromobutoxy)tetrahydropyran in a polar solvent to obtain 5-chloro-2-(4-tetrahydropyran-2-yloxybutyl)-1H-indole of formula (II): 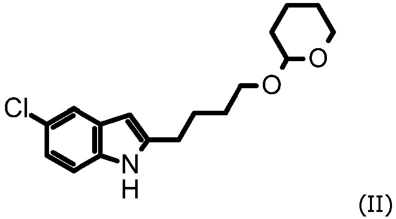
   b) reacting 5-chloro-2-(4-tetrahydropyran-2-yloxybutyl)-1H-indole with 1,3-benzothiazole-6-sulfonyl chloride in a polar solvent to obtain 6-[5-chloro-2-(4-tetrahydropyran-2-yloxybutyl)indol-1-yl]sulfonyl-1,3-benzothiazole of formula (III): 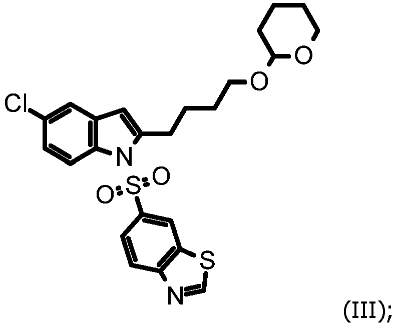
   c) deprotecting the compound of formula (III) to obtain 4-[1-(1,3-benzothiazol-6-ylsulfonyl)-5-chloro-indol-2-yl]butan-1-ol of formula (IV): 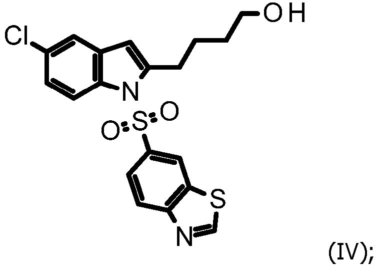 and
   d) oxidizing the compound of formula (IV).
2. The process of item 1, wherein step a) is carried out in the presence of a catalyst.
3. The process of item 2, wherein the catalyst is a palladium-based catalyst is selected from bis(acetonitrile)dichloropalladium (II), palladium diacetate, bis(benzonitrile)dichloropalladium, palladium chloride, and palladium ditrifluoroacetate.
4. The process of item 3, wherein the palladium-based catalyst is bis(acetonitrile)dichloropalladium (II).
5. The process of any one of items 2 to 4, wherein step a) is carried out in the presence of from about 0.05 to about 1.0 equivalent of catalyst based on the starting 5-chloroindole.
6. The process of any preceding items, wherein step a) is carried out in the presence of a base.
7. The process of item 6, wherein step a) is carried out in the presence of from about 1.0 to about 5.0 equivalents of base, based on the starting 5-chloroindole.
8. The process of any one of items 6 to 7, wherein the base is selected from cesium carbonate, sodium carbonate, sodium hydrogencarbonate, potassium carbonate, sodium tert-butylate, potassium tert-butylate, sodium acetate, diisopropylamine, triethylamine, trans-diaminocyclohexane, 1,8-diazabicyclo[5.4.0]undec-7-ene, pyridine, 4-(dimethylamino)pyridine and mixtures thereof.
9. The process of item 8, wherein the base comprises sodium hydrogencarbonate.
10. The process of any preceding item, wherein step a) is carried out at a temperature in the range from about 50°C to about 150°C.
11. The process of item 10, wherein step a) is carried out at a temperature in the range from about 50°C to about 145°C, in particular from about 50°C to about 140°C, from about 50°C to about 135°C, from about 50°C to about 130°C, from about 50°C to about 125°C, from about 50°C to about 120°C, from about 50°C to about 115°C, from about 50°C to about 110°C, from about 50°C to about 105°C, from about 50°C to about 100°C, from about 50°C to about 95°C, from about 50°C to about 90°C, from about 50°C to about 85°C, or from about 50°C to about 80°C.
12. The process of any one of items 10 to 11, wherein step a) is carried out at a temperature in the range from about 50°C to about 80°C, in particular from about 55°C to about 80°, from about 60°C to about 80°C, or-from about 65°C to about 75°C.
13. The process of any preceding item, wherein step a) is carried out for at least about 1 hour and up to about 72 hours.
14. The process of any preceding item, wherein the polar solvent used in step a) is selected from ethyl acetate, acetonitrile, an ether such as methyltetrahydrofuran or dioxane, a water/ether mixture, a water/methanol mixture, toluene, pyridine, sulfolane, tetrahydrothiophene 1,1-dioxyde, N-methyl-2-pyrrolidone, 1,3-dimethyl-3,4,5,6-tetrahydro-2(1H)-pyrimidinone, dimethylsulfoxide and dimethylformamide.
15. The process of any preceding item, wherein step b) is carried out in the presence of from about 0.1 to about 5.0 equivalents of 1,3-benzothiazole-6-sulfonyl chloride, based on the starting 5-chloro-2-(4-tetrahydropyran-2-yloxybutyl)-1H-indole.
16. The process of any preceding item, wherein step b) is carried out at a temperature at or below room temperature.
17. The process of any preceding item, wherein step b) is carried out for at least about 30 minutes and up to about 36 hours.
18. The process of any preceding item, wherein the polar solvent used in step b) is selected from an ether such as methyltetrahydrofuran or dioxane, toluene, pyridine, N-methyl-2-pyrrolidone, 1,3-dimethyl-3,4,5,6-tetrahydro-2(1H)-pyrimidinone, dimethylsulfoxide and dimethylformamide.
19. The process of any preceding item, wherein the same polar solvent is used in step a) and in step b).
20. The process of any preceding item, wherein step c) is carried ou at a temperature from about 15°C to about 30°C.
21. The process of any preceding claim, wherein step c) is carried out for at least 1 hour and up to about 24 hours.
22. The process of any preceding claim, wherein step d) is carried ou at a temperature of no more than about 15°C.
23. The process of any preceding claim, wherein step d) is carried out for at least about 15 minutes and up to about 2 hours.
24. The process of any preceding claim, wherein the polar solvent used in step d) is selected from ethyl acetate, dichloromethane, tetrahydrofuran, acetonitrile, acetone, dimethylsulfoxide and dimethylformamide.

## Claims

1. A process for preparing lanifibranor of formula (I): 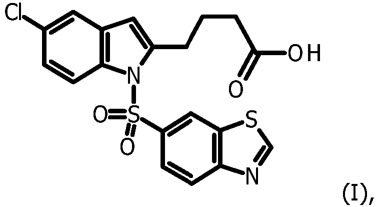 which comprises:
a) reacting 5-chloroindole with 2-(4-bromobutoxy)tetrahydropyran in a polar solvent to obtain 5-chloro-2-(4-tetrahydropyran-2-yloxybutyl)-1H-indole of formula (II): 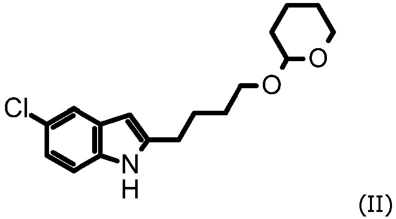
b) reacting 5-chloro-2-(4-tetrahydropyran-2-yloxybutyl)-1H-indole with 1,3-benzothiazole-6-sulfonyl chloride in a polar solvent to obtain 6-[5-chloro-2-(4-tetrahydropyran-2-yloxybutyl)indol-1-yl]sulfonyl-1,3-benzothiazole of formula (III): 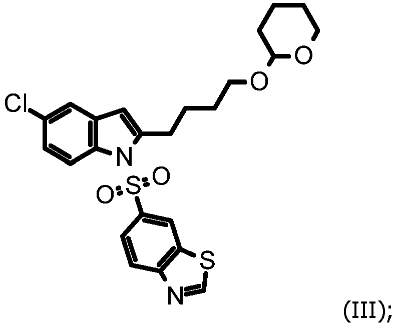
c) deprotecting the compound of formula (III) to obtain 4-[1-(1,3-benzothiazol-6-ylsulfonyl)-5-chloro-indol-2-yl]butan-1-ol of formula (IV): 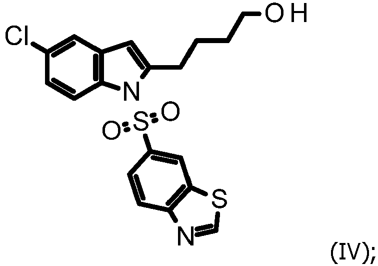 and
d) oxidizing the compound of formula (IV).

2. The process of claim 1, wherein step a) is carried out in the presence of a catalyst.

3. The process of claim 2, wherein the catalyst is a palladium-based catalyst.

4. The process of claim 3, wherein the palladium-based catalyst is selected from bis(acetonitrile)dichloropalladium (II), palladium diacetate, bis(benzonitrile)dichloropalladium, palladium chloride and palladium ditrifluoroacetate.

5. The process of any one of claims 1 to 4, wherein step a) is performed in the presence of a base.

6. The process of claim 5, wherein the base is selected from cesium carbonate, sodium carbonate, sodium hydrogencarbonate, potassium carbonate, sodium tert-butylate, potassium tert-butylate, sodium acetate, diisopropylamine, triethylamine, trans-diaminocyclohexane, 1,8-diazabicyclo[5.4.0]undec-7-ene, pyridine, 4-(dimethylamino)pyridine and mixtures thereof.

7. The process of any one of claims 1 to 6, wherein step a) is carried out at a temperature in the range from about 50°C to about 150°C.

8. The process of any one of claims 1 to 7, wherein the polar solvent used in step a) is selected from ethyl acetate, acetonitrile, an ether such as methyltetrahydrofuran or dioxane, a water/ether mixture, a water/methanol mixture, toluene, pyridine, sulfolane, tetrahydrothiophene 1,1-dioxyde, N-methyl-2-pyrrolidone, 1,3-dimethyl-3,4,5,6-tetrahydro-2(1H)-pyrimidinone, dimethylsulfoxide and dimethylformamide.

9. The process of any one of claims 1 to 8, wherein step b) is carried out at a temperature at or below room temperature.

10. The process of any one of claims 1 to 9, wherein the polar solvent used in step b) is selected from an ether such as methyltetrahydrofuran or dioxane, toluene, pyridine, N-methyl-2-pyrrolidone, 1,3-dimethyl-3,4,5,6-tetrahydro-2(1H)-pyrimidinone, dimethylsulfoxide and dimethylformamide.

11. The process of any one of claims 1 to 10, wherein the same polar solvent is used in steps a) and b).

12. The process of any one of claims 1 to 11, wherein step c) is carried ou at a temperature from about 15°C to about 30°C.

13. The process of any one of claims 1 to 12, wherein step d) is carried ou at a temperature of no more than about 15°C.

14. The process of any one of claims 1 to 13, wherein the polar solvent used in step d) is selected from ethyl acetate, dichloromethane, tetrahydrofuran, acetonitrile, acetone, dimethylsulfoxide and dimethylformamide.
